# EUROPEAN PATENT APPLICATION

(11) **EP 1 129 734 A2**
(43) Date of publication of application: **05.09.2001**
(21) Application number: 01301218.2
(22) Date of filing: 12.02.2001
(51) Int. Cl.: A61M 1/00, A61M 27/00, A61F 13/00

(54) **Apparatus for assisting wound healing**

(30) Priority: 03.03.2000 GB 0005211
(71) Applicant: Mediplus Limited, High Wycombe, Buckinghamshire HP11 2RX (GB)
(72) Inventor: Urie, Robert Graham, Nr. Amersham, Buckinghamshire HP7 0QQ (GB)
(74) Representative: Charig, Raymond Julian

(57) **Abstract**

An apparatus for the treatment of tissue comprises a wound dressing (10) which has a porous layer (11) for contacting a wound site and a lumen tube (20) which includes at least first and second conduits (22,23). A proximal end (21) of the lumen tube is installed into the porous layer of the wound dressing and at least one distal end (40) of the tube provides a first coupling means (35) for coupling the first conduit to a source of vacuum supply and a second coupling means (43) which couples the second conduit to a source of treatment fluid which is beneficial to wound healing. The first and second conduits provide fluid communication between the porous layer (11) and the first and second coupling means. In one arrangement, the proximal end (21) of the tube has a plurality of vacuum delivery orifices (24) extending along a portion of the length of the lumen tube and a plurality of fluid delivery orifices (26) extending along a portion of the length of the lumen tube to provide even distribution of vacuum and fluid to the porous layer.

## Description

The present invention relates to wound healing, and in particular to the use of apparatus for delivering vacuum-assistance to wound sites for the advancement of healing.

In the treatment of open wounds following injury or surgery, the prior art has taught that providing a negative pressure over the wound site can have substantial beneficial effects for promoting healing. For example, WO93/09727 describes that this beneficial effect occurs when the partial vacuum promotes the migration of epithelial and subcutaneous tissue toward the wound site for a time period sufficient to facilitate closure of the wound. In addition, there may be benefits in that the negative pressure reduces bacterial density and that liquid exudates can be conveniently removed from the wound site by the vacuum pump which provides the negative pressure. In WO93/09727 there is described a method and apparatus for treating tissue damage, which includes using a porous dressing, overlying the wound site, having a semi-rigid cup thereover which provides a cavity which is partially evacuated by connection to a vacuum pump.

Various types of pump apparatus have been described in the art, for providing the negative pressure to the wound site, and for safe collection of any exudates from the wound site, for example as described in EP 0853950 A1 and WO97/18007. In each case, a tube is provided connecting the wound site dressing with a vacuum pump via a fluid collection canister.

In particular, WO97/18007 also describes the use of a second tube, preferably incorporated within the same structure as the first tube, running from the wound site to a pressure relief valve and a pressure transducer.

The second tube provides an air passage between the wound site and the pump apparatus for (a) facilitating periodic repressurization of the wound site under automatic control, and (b) enabling pressure monitoring of the wound site and thereby regulation of the pressure at the wound site.

In the present invention, it has been recognized that it would be extremely valuable in promoting healing of the wound to not only provide a suction conduit to a wound site from a pump apparatus, but also to provide a convenient means for delivery of beneficial fluids to the wound site.

According to one aspect, the present invention provides an apparatus for the treatment of tissue comprising:
a wound dressing (10) having a porous layer (11) for contacting or positioning adjacent to a wound site; and
a lumen tube (20) including at least first and second conduits (22,23) therein, wherein a proximal end (21) of the tube is installed into the porous layer of the wound dressing and at least one distal end (40) of the tube provides first coupling means (35) for coupling the first conduit to a source of vacuum supply and second coupling means (43) coupling the second conduit to a source of treatment fluid beneficial to wound healing;
the first and second conduits providing fluid communication between the porous layer (11) and the first and second coupling means.

According to another aspect, the present invention provides an integrated lumen tube (20) for vacuum delivery and fluid delivery to a wound site comprising:
a first conduit (22) extending along a length of the lumen tube;
a second conduit (23) extending along a length of the lumen tube;
a proximal end (21) having a plurality of vacuum delivery orifices (24) extending along a portion of the length of the lumen tube, each vacuum delivery orifice passing through the wall of the lumen tube to the first conduit (22), and a plurality of fluid delivery orifices (26) extending along a portion of the length of the lumen tube, each fluid delivery orifice passing through the walls of the lumen tube to the second conduit (23); and
first coupling means (35) for coupling the first conduit to a source of vacuum supply and second coupling means (43) for coupling the second conduit to a source of fluid supply.

According to another aspect, the present invention provides a lumen tube (20) for vacuum delivery and fluid delivery to a wound site comprising:
a first conduit (22) extending along a length of the lumen tube;
a second conduit (23) extending along a length of the lumen tube;
a proximal end (21) having at least one vacuum delivery orifice (24) communicating with the first conduit (22), and at least one fluid delivery orifice (26) communicating with the second conduit (23); and
first coupling means (35) for coupling the first conduit to a source of vacuum supply and second coupling means (43) for coupling the second conduit to a source of fluid supply, the second coupling means being provided on a second distal end of the lumen tube provided on a branch (41) which diverges from the main body of the lumen tube at an intermediate point between the proximal end and the first distal end.

Embodiments of the present invention will now be described by way of example and with reference to the accompanying drawings in which:
Figure 1 shows a schematic diagram of a combined vacuum and therapeutic fluid delivery system according to the present invention;
Figure 2 shows a radial cross-sectional diagram of a delivery tube used in figure 1;
Figure 3 shows a radial cross-sectional view of a proximal end of the delivery tube of figure 1; and
Figure 4 shows a longitudinal cross-sectional view of a proximal end of an alternative delivery tube to that of figure 1.

In the present invention it has been realised that, in wound healing, it can be beneficial to deliver therapeutic fluids to the wound site in conjunction with the provision of vacuum-assisted healing. Such fluids may be for irrigation purposes, for delivery of drugs or infusions or for other beneficial effects. The fluid delivery to the wound site need not be a liquid: in certain circumstances, delivery of a pure oxygen supply to the wound site has been found to be beneficial.

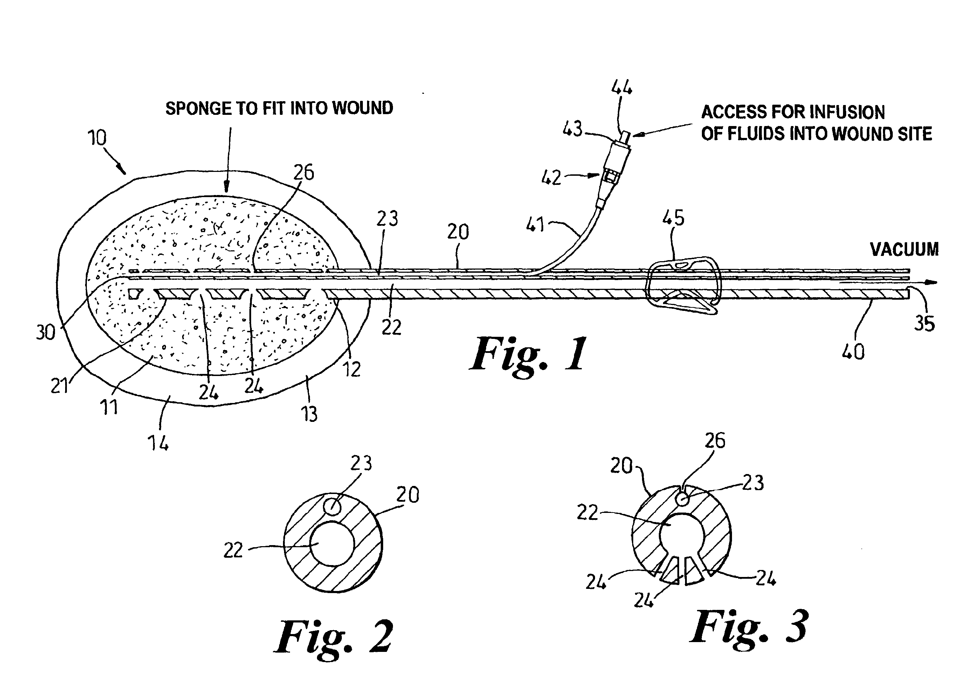

Referring to figure 1, a dressing 10 is provided for covering a wound site. The dressing may be of conventional construction as described in the art, comprising a sponge layer 11 for fitting into a wound cavity or generally over a wound site. The sponge layer is generally porous to fluids, both liquid and gaseous, and includes a cavity 12 for receiving a lumen tube 20 to be described hereinafter. Overlying and overlapping an outer surface of the sponge layer 11 is non-porous covering 13 which can be self-adhesive around its periphery 14 to provide means for sealingly engaging the sponge layer onto or into the wound site by adhesion with the skin adjacent to the wound site. The covering 13 also prevents escape of fluids delivered to, or exuded from, the wound site, and enables the maintenance of a partial vacuum over or in the wound site. Various constructions of dressing 10 incorporating vacuum delivery tubes are well known in the art and will not be discussed further here.

A multi-lumen tube 20 is inserted into the cavity 12 in the sponge layer 11 and is retained therein by suitable adhesive or other available mechanism.

The lumen tube is preferably of the double lumen variety having a cross-sectional profile as shown in figure 2. It will be understood, however, that other types of multi-lumen tube could be used, including those of the coaxial variety where multiple conduits are provided down the outside of a central conduit.

In the preferred embodiment, the double lumen tube 20 is provided as a 7 mm diameter PVC tube having a large central conduit 22 with a smaller peripheral conduit 23. At the proximal end 21 of the double lumen tube 20 the tube is provided with one or more vacuum delivery orifices 24 which extend radially outward from the central conduit 22 and one or more fluid delivery orifices 26 which extend radially outward from the peripheral conduit 23. The vacuum delivery orifices 24 may be provided at one radial angle or at several radial angles as shown in figure 3.

As shown in figure 1 and figure 3, the vacuum delivery orifices 24 and fluid delivery orifices 26 are preferably spatially separated from one another to prevent fluid being delivered directly or close to a site of vacuum delivery and thus causing immediate removal of delivered fluids. In the embodiment shown in figure 3, the spatial separation of fluid delivery orifices 26 and vacuum delivery orifices 24 is achieved by means of circumferential separation. In other words, as shown, the vacuum delivery orifices 24 are provided in one side of the tube 20 and the fluid delivery orifices 26 are provided at an approximately diametrically opposed side of the tube. This ensures that any direct path between vacuum delivery orifices and fluid supply orifices is occluded by the lumen tube itself. As shown in figure 1, the orifices 24 and 26 are preferably distributed approximately regularly along the length of the proximal end 21 of the tube 20 residing in sponge layer 11. In another embodiment, the orifices 24 and/or 26 might be spaced closer to one another at an end most remote from the source of vacuum and fluids to compensate for pressure gradients in the lumen tube.

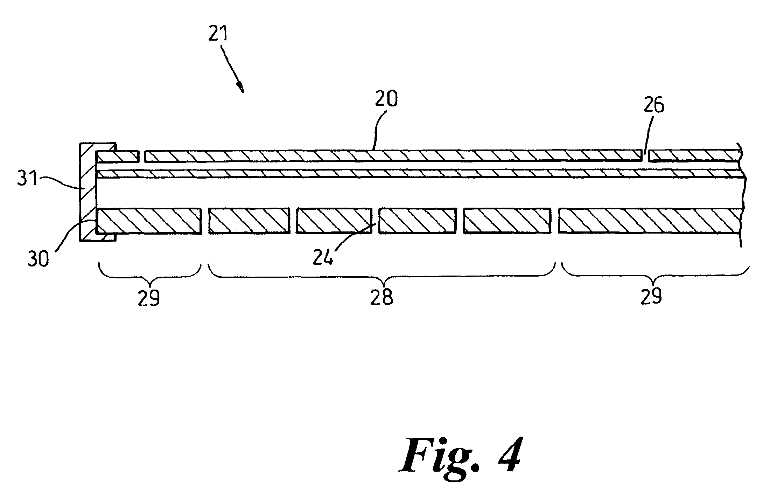

In an alternative arrangement, as shown in figure 4, the spatial separation of fluid delivery orifices 26 and vacuum delivery orifices 24 may be achieved by means of longitudinal separation along the length of the proximal end 21 of tube 20, the longitudinal separation being at least of the order of several times the diameter of the orifices. In the arrangement shown, the vacuum delivery orifices 24 are situated at regular intervals along a central part 28 of the proximal end 21 of tube 20, while the fluid delivery orifices are located at longitudinal extremities 29 of the proximal end 21. In this configuration, the vacuum delivery orifices and fluid delivery orifices may be situated at any or multiple radial locations around the tube 20 circumference and need not be diametrically opposed from one another.

In an alternative configuration (not shown), the vacuum delivery orifices and fluid delivery orifices may be spaced longitudinally from one another at regular spacing and alternating positions to provide an even distribution of delivered fluid and vacuum along the length of the proximal end 21.

The end 30 of the tube 20 may be capped with a sealing cap 31 to prevent egress of fluids and/or vacuum delivery from the very end of the tube. Alternatively, the end 30 may be plugged or fused closed by heat treatment. The vacuum delivery orifices 24 and the fluid delivery orifices 26 may be of the same size or, as shown, the vacuum delivery orifices are preferably larger than the fluid delivery orifices. The diameter of the orifices may also be varied, for example according to the distance of the orifice from the vacuum source or fluid source. This feature can be used to assist in maintaining an even distribution of fluid and vacuum delivery to the wound dressing, offsetting any pressure differential created in the proximal end 21 of the tube 20.

With further reference to figure 1, a vacuum connection is made to a distal end 40 of the tube 20, for example using a vacuum pump and collection chamber apparatus as well known in the art discussed *supra*. To this end, the distal end 40 is provided with a coupling means 35 which may be as simple as a push fit of the flexible lumen tube onto a vacuum supply or suitable connector device.

A fluid delivery system for use with the present invention might be integrated into vacuum pump and collection chamber apparatus if desired. However, in the preferred embodiment as shown in figure 1, the multi-lumen tube is provided with a branch portion 41 which diverges from the main tube walls at some intermediate point between the proximal end 21 and the distal end 40. The branch portion 41 provides a continuous conduit with the peripheral conduit 23 leading to the proximal end 21. The peripheral conduit 23 leading to the distal end 40 beyond the point of divergence may be occluded or simply omitted altogether.

The branch portion 41 is preferably provided, at a distal end thereof, with an integral in-line one-way valve 42 and a coupling member 43 provided with a male connector 44 for connecting to a conventional infusion or fluid supply device as is well known in the art.

It will be understood that the delivery of vacuum and fluids to the wound site via the apparatus of the present invention will be according to desired clinical practice for a given clinical condition. For example, the clinical requirement for the treatment of burns may be different from a wound generated by surgery. The coupling member 43 allows for a continuous drip feed of fluids to the fluid delivery orifices 26, or the delivery may be intermittent and manually administered by, for example, syringe. The vacuum delivery may be continuous or intermittent and control may be provided manually or automatically according to the type of vacuum delivery apparatus provided. The delivery of fluids to the wound site and the delivery of vacuum to the wound site may be contemporaneous, alternating, or otherwise intermittent, according to the treatment schedule. In the event that automatic control of vacuum supply is not provided, or for prevention of leakage from the tube 20 during changing of a canister, the main body of the tube 20 may be provided with a closure clamp 45 for closing the main conduit 22 separate from the peripheral conduit 23 leading to the branch portion 41.

In the preferred embodiment, the flexible nature of the multi-lumen tube 20 enables the proximal end to be inserted into a shaped dressing 10, for example to curve along a cavity 12 which extends around the area of the dressing 10 for improved coverage of the wound area.

It will be understood that various modifications and adaptations of the specific embodiments described herein may be made without departing from the scope of the invention as defined with reference to the accompanying claims.

## Claims

1. Apparatus for the treatment of tissue comprising:
a wound dressing (10) having a porous layer (11) for contacting or positioning adjacent to a wound site; and
a lumen tube (20) including at least first and second conduits (22,23) therein, wherein a proximal end (21) of the tube is installed into the porous layer of the wound dressing and at least one distal end (40) of the tube provides first coupling means (35) for coupling the first conduit to a source of vacuum supply and second coupling means (43) coupling the second conduit to a source of treatment fluid beneficial to wound healing;
the first and second conduits providing fluid communication between the porous layer (11) and the first and second coupling means.

2. Apparatus according to claim 1 in which the lumen tube (20) comprises a flexible tube having a central conduit (22) extending therethrough and a peripheral conduit extending therethrough, alongside the central conduit.

3. Apparatus according to claim 2 in which the central conduit (22) is of greater cross-sectional area than the peripheral conduit (23).

4. Apparatus according to claim 1 or claim 2 in which the second coupling means (43) is provided on a second distal end provided on a branch (41) which diverges from the main body of the lumen tube.

5. Apparatus according to claim 4 in which the second coupling means (43) includes a one-way valve (42).

6. Apparatus according to claim 1 in which the proximal end (21) of the lumen tube (20) further includes a plurality of vacuum delivery orifices (24) extending along a portion of the length of the lumen tube, each orifice passing through the wall of the lumen tube to the first conduit (22).

7. Apparatus according to claim 1 in which the proximal end (21) of the lumen tube (20) further includes a plurality of fluid delivery orifices (26) extending along a portion of the length of the lumen tube, each orifice passing through the walls of the lumen tube to the second conduit (23).

8. Apparatus according to claim 6 and claim 7 in which each of said vacuum delivery orifices (24) is spatially separated from each of said fluid delivery orifices (26).

9. Apparatus according to claim 8 in which each of said vacuum delivery orifices (24) is provided substantially on one side of the lumen tube (20) and each of said fluid delivery orifices (26) is provided substantially on an opposite side of the lumen tube.

10. Apparatus according to claim 8 in which said vacuum delivery orifices (24) extend longitudinally over a central portion (28) of the proximal end (21) of lumen tube (20), and the fluid delivery orifices are located at longitudinal extremities (29) of the proximal end.

11. Apparatus according to claim 8 in which said vacuum delivery orifices (24) and said fluid delivery orifices (26) are longitudinally spatially separated in an alternating pattern along the proximal end of the lumen tube (20).

12. An integrated lumen tube (20) for vacuum delivery and fluid delivery to a wound site comprising:
a first conduit (22) extending along a length of the lumen tube;
a second conduit (23) extending along a length of the lumen tube;
a proximal end (21) having a plurality of vacuum delivery orifices (24) extending along a portion of the length of the lumen tube, each vacuum delivery orifice passing through the wall of the lumen tube to the first conduit (22), and a plurality of fluid delivery orifices (26) extending along a portion of the length of the lumen tube, each fluid delivery orifice passing through the walls of the lumen tube to the second conduit (23); and
first coupling means (35) for coupling the first conduit to a source of vacuum supply and second coupling means (43) for coupling the second conduit to a source of fluid supply.

13. The lumen tube of claim 12 in which the first coupling means (35) is provided at a first distal end (40) of the lumen tube and in which the second coupling means is provided at a second distal end of the lumen tube on a branch (41) which diverges from the main body of the lumen tube at an intermediate point between the proximal end and the first distal end.

14. The lumen tube of claim 12 or claim 13 in which the lumen tube (20) comprises a flexible tube having a central conduit (22) extending therethrough and a peripheral conduit extending therethrough, alongside the central conduit.

15. The lumen tube of claim 14 in which the central conduit (22) is of greater cross-sectional area than the peripheral conduit (23).

16. The lumen tube of claim 13 in which the second coupling means (43) includes a one-way valve (42).

17. The lumen tube of claim 12 in which each of said vacuum delivery orifices (24) is spatially separated from each of said fluid delivery orifices (26).

18. The lumen tube of claim 17 in which each of said vacuum delivery orifices (24) is provided substantially on one side of the lumen tube (20) and each of said fluid delivery orifices (26) is provided substantially on an opposite side of the lumen tube.

19. The lumen tube of claim 17 in which said vacuum delivery orifices (24) extend longitudinally over a central portion (28) of the proximal end (21) of lumen tube (20), and the fluid delivery orifices are located at longitudinal extremities (29) of the proximal end.

20. The lumen tube of claim 17 in which said vacuum delivery orifices (24) and said fluid delivery orifices (26) are longitudinally spatially separated in an alternating pattern along the proximal end of the lumen tube (20).

21. A lumen tube (20) for vacuum delivery and fluid delivery to a wound site comprising:
a first conduit (22) extending along a length of the lumen tube;
a second conduit (23) extending along a length of the lumen tube;
a proximal end (21) having at least one vacuum delivery orifice (24) communicating with the first conduit (22), and at least one fluid delivery orifice (26) communicating with the second conduit (23); and
first coupling means (35) for coupling the first conduit to a source of vacuum supply and second coupling means (43) for coupling the second conduit to a source of fluid supply, the second coupling means being provided on a second distal end of the lumen tube provided on a branch (41) which diverges from the main body of the lumen tube at an intermediate point between the proximal end and the first distal end.
